(19) Europäisches Patentamt
European Patent Office
Office européen des brevets

(11) Veröffentlichungsnummer: **0 309 983**
**A2**

(12) # EUROPÄISCHE PATENTANMELDUNG

(21) Anmeldenummer: **88115873.7**

(51) Int. Cl.⁴: **C07K 1/02 , C07K 5/00**

(22) Anmeldetag: **27.09.88**

(30) Priorität: **01.10.87 DE 3733198**

(43) Veröffentlichungstag der Anmeldung:
**05.04.89 Patentblatt 89/14**

(84) Benannte Vertragsstaaten:
**AT BE CH DE FR GB IT LI LU NL SE**

(71) Anmelder: **Kernforschungsanlage Jülich Gesellschaft mit beschränkter Haftung**
**Postfach 1913**
**D-5170 Jülich 1(DE)**

(72) Erfinder: **Schütz, Hans-Jürgen**
**Stintenberger Strasse 54**
**D-4020 Mettmann(DE)**
Erfinder: **Wandrey, Christian, Prof.**
**Wolfshovener Strasse 139**
**D-5170 Jülich(DE)**

(54) **Enzymatisches Verfahren zur Herstellung von Peptiden, insbesondere Dipeptiden und Vorrichtung dafür.**

(57) Ggf. N-geschützte Peptide, insbesondere Dipeptide, werden durch kontinuierliche enzymatische Umsetzung von N-geschütztem Aminosäurealkylester in Gegenwart von Serinprotease oder Peptidase, insbesondere von Carboxypeptidasen, in wäßriger Phase unter Ausschluß organischer Lösungsvermittler und unter Zurückhaltung des Enzyms im Reaktionsraum mit einem solchen Überschuß an freier Aminosäure (Molverhältnis > 50 : 1) und einer solchen Verweilzeit erhalten, die zur quantitativen Umwandlung des Esters unter Bildung des N-geschützten Peptids führen, das von der vom Reaktionsraum abgehenden Reaktionsmischung durch Adsorption an einem hydrophoben Adsorber abgetrennt wird, während der aminosäurehaltige Rest der Reaktionsmischung zum Reaktionsraum zurückgeht. Vom Adsorber wird das N-geschützte Peptid mit wäßriger Lösung eluiert und dann enzymatisch desacyliert. Peptidbildung und Schutzgruppenabspaltung erfolgen vorzugsweise im Enzymmembranreaktor oder mit träger-fixiertem Enzym. Als N-geschützter Aminosäureester dient insbesondere N-Phenacetyl-aminosäurealkylester, dessen Schutzgruppe enzymatisch mit Penicillin-G-Acylase abgespalten wird. Besonders untersucht wurde die Umsetzung von N-geschütztem aromatischen Aminosäureester mit freier aliphatischer Aminosäure (insbesondere Bildung von TyrAla).

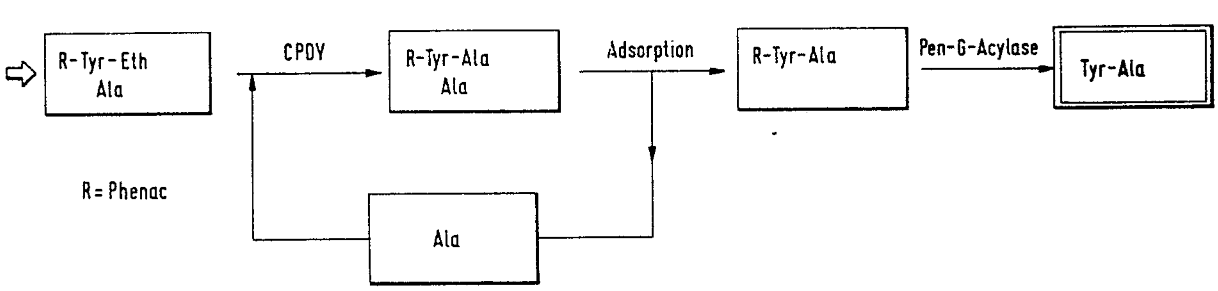


FIG. 1

EP 0 309 983 A2

Xerox Copy Centre

## Enzymatisches Verfahren zur Hestellung von Peptiden, insbesondere Dipeptiden und Vorrichtung dafür

Die Erfindung bezieht sich auf ein Verfahren zur Herstellung von ggf. N-geschützten Peptiden, insbesondere Dipeptiden, durch enzymatische Umsetzung von N-geschütztem Aminosäurealkylester mit in hohem Überschuß verwendeter Aminosäure in wässriger Phase in Gegenwart von Serinprotease oder Peptidase und enzymatische Abspaltung der Schutzgruppe vom gebildeten Peptid sowie aufeine dafür geeignete Vorrichtung.

Di- und Oligopeptide, wie z. B. Tyr-Ala, werden möglicherweise in Zukunft eine bedeutende Rolle in der parenteralen Ernährung spielen: sie verursachen eine geringe Osmolarität und sind ausgezeichnet wasserlöslich und damit für Infusionslösungen gut brauchbar. Ferner zeichnen sich Peptide zum Teil durch hochinteressante pharmakologische Eigenschaften aus, wie etwa die Peptid-Hormone (z. B. Tyr-Arg). Ihre Zulassung als pharmazeutische Mittel erfordert allerdings eine hohe Reinheit, insbesondere Enantiomerenreinheit von mehr als 99,9 ee. Solche hohen Enantiomerenreinheiten sind bei herkömmlichen chemischen Verfahren nur mit hohem Aufwand zu erreichen. Die enzymatische Peptidsynthese erscheint daher besonders attraktiv.

Bekannt ist aus der US-PS 4 339 534 ein Verfahren zur Herstellung von N-geschützten (N-acylierten) Dipeptiden durch enzymatische Umsetzung von N-Acylaminosäurealkylestern (A) mit gegebenenfalls C-ge schützter Aminosäure (B) unter Verwendung von Carboxypeptidase als Enzym, die insbesondere in immobilisierter Form (trägerfixiert) verwendet wird.

Aus den Beispielen der US-PS entnimmt man die Nützlichkeit eines organischen Lösungsvermittlers (üblicherweise Alkohol) sowie die Verwendung von stark überschüssiger Aminosäure im Vergleich zum Ester. Weiter zeigen die mitgeteilten Ergebnisse eine deutliche Verbesserung, wenn die Aminosäurekomponente B in Form von Amid und nicht als freie Säure verwendet wird (was allerdings eine zusätzliche Schutzgruppenabtrennung nötig macht, um zum Peptid selbst zu gelangen).

Detaillierte Angaben über die Abspaltung der Schutzgruppen, insbesondere der N-Acyl-gruppe vom N-geschützten Dipeptid sind in der US-PS nicht enthalten.

Die Beispiele skizzieren eine batch-weise Herstellung mit chromatographischer Auftrennung der Reaktionsmischung am Ende.

Nähere Einzelheiten über eine Verfahrensweise zur Herstellung von N-Acyl-dipeptid-amid am Beispiel der Umsetzung von N-Acyl-arginin mit Methioninamid in Gegenwart von immobilisierter Carboxypeptidase werden von S. M. Cramer u. a. im J. Chromat. 394 (1987) 305 - 314 angegeben, die eine Peptidbildung im Festbettreaktor mit umgepumpter Reaktionsmischung und Aufarbeitung des Produktes nach ausreichender Umsetzung durch Verdrängungschromatographie der in einem Zwischenbehälter auf pH 2,5 gebrachten Reaktionsmischung im Batchbetrieb beschreiben.

Als N-Schutzgruppen werden gemäß der o. g. US-PS vornehmlich Benzoyl-, Acetyl- und Benzyloxycarbonyl aus einer Mehrzahl von als möglich angegebenen Gruppen verwendet. Von F. Widmer u. a. ("Enzymatische Peptidsynthese" in "Peptides 1982" Walter de Gruyter & Co, Berlin New York, 1983, Seiten 375 bis 379) wird aber auch über die Verwendung der N-Phenacetyl-Gruppe als Schutzgruppe für die Umsetzung von N-Acyl-aminosäurealkylester mit Aminosäureamid in Gegenwart von Carboxypeptidase Y berichtet und auf eine eventuell mögliche enzymatische Abspaltbarkeit der Schutzgruppe mit Penicillin-Acylase hingewiesen.

C. Fuganti und P. Grasselli berichten in Tetrahedron Letters, 27 (1986), Seiten 3191 - 3194 über die erfolgreiche enzymatische Desacylierung von N-Phenacetyl-dipeptid-ester, d. h. von einem C-geschützten N-Acyldipeptid mittels an Eupergit immobilisierter Penicillin-Acylase während die Schutzgruppe von der N-acylierten Aminosäure selbst nicht abgespalten wurde.

Ziel der Erfindung ist nun eine Erhöhung der Wirtschaftlichkeit der Peptidsynthese durch kontinuierliche Arbeitsweise, wobei insbesondere auf einen minimalen Enzymverbrauch geachtet und durch Verwendung von Aminosäure als Komponente B die zusätzliche Abtrennung der C-Schutzgruppe vermieden werden soll.

Das zu diesem Zweck entwickelte erfindungsgemäße Verfahren der eingangs genannten Art ist dadurch gekennzeichnet, daß man die Umsetzung kontinuierlich unter Ausschluß organischer Lösungsvermittler und mit im Reaktionsraum zurückgehaltenem Enzym, mit einem solchen Aminosäureüberschuß und einer solchen Verweilzeit ablaufen läßt, die zur quantitativen Umwandlung des Aminosäureesters führt, daß man das N-geschützte Peptid durch Adsorption an einem hydro phoben Adsorber von der aus dem Reaktionsraum abgehenden Reaktionsmischung abtrennt, die zur Umwandlung zurückgegeben wird und daß man schließlich ggf. die Schutzgruppe von dem mit wässriger Lösung vom Adsorber eluierten N-geschützten Peptid enzymatisch abspaltet.

Auf diese Weise können ggf. N-geschützte Dipeptide bzw. Oligopeptide in wirtschaftlicher Weise erhalten werden.

Das Enzym wird dabei insbesondere in trägerfixierter Form (z. B. an Eupergit ein hochmolekularer Träger mit Epoxygruppen; allgemein können hochmolekulare Träger mit für die Proteinanlagerung chemisch reaktiven Gruppen vorgesehen werden) oder in einem Enzymmembranreaktor angewandt.

Für eine Abtrennung des gebildeten N-geschützten (Di)Peptids von der Reaktionsmischung dient insbesondere Amberlite XAD-2.

Das Molverhältnis von Aminosäure zu N-geschütztem Aminosäureester liegt insbesondere bei > 50 : 1, vorzugsweise bei ≥200 : 1 abhängig von den speziell umzusetzenden Komponenten. Für die Umsetzung von Ala mit Phenac-Tyr-OMe wurde ein Verhältnis von etwa 500 : 1 mit Erfolg angewandt.

Als Enzyme für die Peptidbildung dienen insbesondere Carboxypeptidasen, speziell Carboxypeptidase Y.

Der N-geschützte Aminosäurealkylester (Komponente A) ist insbesondere ein N-Acyl-, speziell ein Phenacetylaminosäurealkylester und zur enzymatischen Abspaltung der Schutzgruppe wird insbesondere Penicillin-G-Acylase (speziell im Enzymmembranreaktor oder in trägerfixierter Form) verwendet.

Als N-Phenacetylaminosäurealkylester dient insbesondere ein Ester einer aromatischen Aminosäure und insbesondere N-Phenacetyltyrosinniederalkylester (wobei Niederalkyl insbesondere 1 bis 4 C-Kohlenstoffatome umfassen soll).

Als Komponente B wurden erfolgreich Alanin, Serin, Glycin, Methionin, Isoleucin, Valin, Leucin sowie Arginin und insbesondere Alanin verwendet.

Speziell untersucht wurde die LL-Dipeptidbildung zwischen einer gutlöslichen aliphatischen und einer schlechtlöslichen aromatischen Aminosäure am Beispiel Tyrosin, Alanin. Die nachfolgenden Reaktionsgleichungen zeigen die durchgeführte Umsetzung:

$$\text{(I)}\quad \text{Phenac-Tyr-OMe+Ala} \xrightarrow[\text{CPDY}]{H_2O} \text{Phenac-TyrAla+Phenac-Tyr+MeOH}$$

$$\text{(II)}\quad \text{Phenac-Tyr+Phenac-TyrAla} \xrightarrow[\text{Pen-G-Acylase}]{H_2O} \text{Phenac+Tyr +TyrAla}$$

Die vorstehenden Reaktionen laufen in wässriger Phase ab und wurden kontinuierlich durchgeführt.

Die Peptidbildung (I) wurde sowohl im Enzymmembranreaktor (EMR; siehe z. B. EP-A-23 346 und 24 547) als auch mit an einem Träger fixiertem immobilisierten Enzym durchgeführt. Als Enzym wurde CPDY der Firma Carlbiotech verwendet. Für die Fixierung diente Eupergit (ein mit Oxiranringen versehener Polymerträger). Es wurde mit Verweilzeiten bis unter 10 min. gearbeitet, die in Abhängigkeit von der Enzymkonzentration vorab ermittelt wurden. Die Enzymdesaktivierung lag bei ca. 0,7 %/d. Es zeigte sich, daß die Selektivität der Reaktion bei homogener Katalyse (im Enzymmembranreaktor) leicht erhöht war (um 5 bis 10 %).

Das bei der Umsetzung (I) gewonnene N-acylierte Dipeptid wurde von der Reaktionsmischung durch Adsorption an einem hydrophoben Adsorber, wie z. B. Amberlite XAD-2, abgetrennt, an dem das N-Acyldipeptid aus dem EMR-Ablauf adsorbiert wird, ohne daß irgendeine pH-Änderung notwendig wäre. Für kontinuierlichen Betrieb werden zweckmäßigerweise zwei Amberlite-Säulen umschichtig verwendet, von denen die eine mit EMR-Ablauf beaufschlagt wird, während aus der anderen das in Phase 1 akkumulierte N-Acyl-dipeptid eluiert wird.

Die im großem Überschuß eingesetzte Aminosäure wird vom Adsorber nicht festgehalten und zur Umsetzung rückgeführt.

Die Abspaltung der Schutzgruppe (Umsetzung II) mit Penicillin-G-Acylase erfolgt zweckmäßigerweise bei pH-Werten von 7 bis 8,5, insbesondere um 7,5. Auch diese Reaktion kann sowohl mit trägerfixiertem Enzym als auch in homogener Phase im EMR durchgeführt werden. Als besonders geeignet erwies sich die Umsetzung mit an Eupergit immobilisiertem Enzym. Das erhaltene Dipeptid kann aus der Reaktionsmischung, z. B. durch Kristallisation gewonnen und gereinigt werden.

Die angefügten Zeichnungen veranschaulichen die durchgeführte Umsetzung; es zeigen

Figur 1 ein Reaktionsschema und

Figur 2 ein Schema für die kontinuierlich arbeitende Apparatur zur Dipeptidherstellung

Die in Figur 2 dargestellte Apparatur mit Eingang bei a und Ausgang bei b umfaßt ein Ausgleichs- und

Substratvorratsgefäß 1, das mit Ausgangsmaterial beschickt wird und rezykliertes Alanin (nach Entfernung des Alkohols durch Gasstrippen) erhält. Von diesem aus wird über ein Primärfilter 2 mittels einer Dosierpumpe 3 über ein Sterilfilter 4 Reaktionsflüssigkeit in den Enzymmembranreaktor 5 für die Peptidsynthese gepumpt, der bei 25 °C arbeitet.

Der Ablauf des Reaktors läuft über eine der abwechselnd zur Produktabtrennung betriebenen Säulen 6, wo das gebildete N-Acyldipeptid bis zum Durchbruch auf der Säule angesammelt wird, während das im Ablauf der Säule befindliche Alanin zum Ausgang zurückläuft über ein Polarimeter 7, mit dessen Hilfe die Alaninrestkonzentration bestimmt wird.

Aus einem Vorrat 8 wird Spülflüssigkeit (verdünnte Salzsäure) nach Umschaltung der Säulen in die vollständig beladene Säule gepumpt und nachfolgend aus 9 Elutionsflüssigkeit (verdünnte NaOH).

Die Elutionsflüssigkeit gelangt in das Gefäß 10, das als Ausgleichsgefäß wirkt und in dem der pH-Wert in den für die Desacylierung geeigneten Bereich gebracht wird.

Pumpe und Sterilfilter sind wiederum mit 3 bzw. 4 bezeichnet. P sind Druckaufnehmer. Die Desacylierung findet schließlich im Enzymmembranreaktor 11 bei 25 °C statt, aus dessen Ablauf das Dipeptid ohne Schwierigkeit von der anwesenden Phenylessigsäure und dem von der Hydrolyse in der Synthesestufe I herrührenden Tyrosin durch selektive Adsorptionen oder Kristallisation abgetrennt werden kann.

Nachfolgend wird das erfindungsgemäße Verfahren anhand eines praktischen Beispiels näher erläutert:

## Beispiel

Ein 10 ml EMR wurde mit 1 mg CPDY bei 25 °C betrieben. Die Verweilzeit betrug 10 Minuten. Die Konzentration der Substratlösung entsprach 2 mM Phenac-Tyr-OMe und 1 M Alanin. Ihr pH-Wert lag bei 8,4. Die aus dem EMR abgezogene Produktlösung enthielt 65 % N-acyliertes Dipeptid und 35 % Hydrolysat N-Phenac-Tyr (bezogen auf den eingesetzten Ester) bei einer Raum-Zeit-Ausbeute von 50,6 g/ld. Die Enzymdesaktivierung lag bei 0,7 % pro Tag, d. h. es wurden 3,4 mg Enzym pro kg Dipeptid verbraucht. Das N-Acyldipeptid wurde durch Adsorption an Amberlite XAD-2 abgetrennt. Das nicht umgesetzte Alanin wurde wieder mit Phenac-Tyr-OMe versetzt und in den Reaktor zurückgeführt.

Das adsorbierte N-Acyl-dipeptid wurde vom Amberlite mit 50 mM NaOH desorbiert, die Lösung mit verdünnter HCl auf pH 7,7 gebracht und die Schutzgruppe Phenac in wässriger Lösung mit Hilfe von an Eupergit immobilisierter Penicillin-G-Acylase (10 mg Enzym/1 g Eupergit) bei 25 °C abgespalten. Die Abspaltung war vollständig, wenn die Verweilzeit in der Säule bei 55 Min lag. Die Verweilzeit wird üblicherweise im Bereich von 40 - 70 Min gewählt, wobei zu lange Verweilzeiten wegen der dann zunehmenden Hydrolyse des Dipeptids vermieden werden sollten. Im Enzymmembranreaktor konnten mit vergleichbaren Enzymkonzentrationen Verweilzeiten bis herab zu 10 Min erreicht werden.

Die im Ablauf enthaltene Phenylessigsäure wurde durch Adsorption an Amberlite XAD-2 aus der Produktlösung entfernt.

Aufgearbeitet wurde durch fraktionierte Kristallisation. Die optische Reinheit des erhaltenen Dipeptids lag bei > 99 ee (Nachweisgrenze).

Alternativ wurde die Desacylierung im Enzymmembranreaktor durchgeführt:

Dazu wurde eine 2 mM N-Acyldipeptidlösung (Ablauf der Amberlite XAD-2 Säule) auf pH 7,8 eingestellt und mit einer Verweilzeit von 15 Min durch einen 10 ml EMR mit einer Membran mit einem cut-off von 5000 gepumpt. In dem Reaktor befanden sich 20 mg Penicillin-G-Acylase. Der maximale Umsatz lag bei 85 %. Die Enzymdesaktivierung lag bei 1,3 % pro Tag.

Obwohl im Vorstehenden vornehmlich die Herstellung von freien Dipeptiden beschrieben wird, können erfindungsgemäß selbstverständlich auch N-geschützte Peptide bzw. Dipeptide erhalten werden, die als Zwischenprodukte oder auch ggf. selbst mit eigener pharmakologischer Wirkung begabt von Interesse sein können.

Für die Herstellung von Oligopeptiden wird selbstverständlich statt der freien Aminosäure freies Peptid und/oder statt des N-geschützten Aminosäureesters ein N-geschützter Peptidester verwendet.

## Ansprüche

1. Verfahren zur Herstellung von ggf. N-geschützten Peptiden, insbesondere Dipeptiden, durch enzymatische Umsetzung von N-geschütztem Aminosäurealkylester mit in hohem Überschuß verwendeter Aminosäure in wässriger Phase in Gegenwart von Serin-protease oder Peptidase und enzymatische Abspaltung der Schutzgruppe vom gebildeten Peptid,

**dadurch gekennzeichnet,**
daß man die Umsetzung kontinuierlich unter Ausschluß organischer Lösungsvermittler und mit im Reaktionsraum zurückgehaltenem Enzym, mit einem solchen Aminosäureüberschuß und einer solchen Verweilzeit ablaufen läßt, die zur quantitativen Umwandlung des Aminosäureesters führt, daß man das N-geschützte Peptid durch Adsorption an einem hydrophoben Adsorber von der aus dem Reaktionsraum abgehenden Reaktionsmischung abtrennt, die zur Umwandlung zurückgegeben wird und daß man schließlich ggf. die Schutzgruppe von dem mit wässriger Lösung vom Adsorber eluierten N-geschützten Peptid enzymatisch abspaltet.

2. Verfahren nach Anspruch 1,
**dadurch gekennzeichnet,**
daß die Peptidbildung im Enzymmembranreaktor oder mit an einem Träger, insbesondere Eupergit, fixiertem Enzym erfolgt.

3. Verfahren nach Anspruch 1 oder 2,
**dadurch gekennzeichnet,**
daß die Abtrennung des N-geschützten Peptids von der Reaktionsmischung durch Adorption an Amberlite XAD-2 erfolgt.

4. Verfahren nach einem der vorangehenden Ansprüche,
**dadurch gekennzeichnet,**
daß mit einem Molverhältnis von Aminosäure : N-geschütztem Aminosäureester von > 50 : 1, insbesondere von größenordnungsmäßig 500 : 1 gearbeitet wird.

5. Verfahren nach einem der vorangehenden Ansprüche,
**dadurch gekennzeichnet,**
daß die Peptidbildung in Gegenwart von Carboxypeptidasen, insbesondere Carboxypeptidase Y stattfindet.

6. Verfahren nach einem der vorangehenden Ansprüche,
**dadurch gekennzeichnet,**
daß der N-geschützte Aminosäurealkylester ein N-Acyl-, insbesondere N-Phenacetylaminosäurealkylester ist und zur enzymatischen Abspaltung der Schutzgruppe Penicillin-G-Acylase verwendet wird.

7. Verfahren nach Anspruch 6,
**dadurch gekennzeichnet,**
daß die enzymatische Desacylierung in wäßriger Phase mit trägerfixierter Penicillin-G-Acylase oder im Enzymmembranreaktor erfolgt.

8. Verfahren nach Anspruch 6,
**dadurch gekennzeichnet,**
daß als N-Phenacetylaminosäurealkylester ein Ester einer aromatischen Aminosäure und insbesondere N-Phenacetyl-tyrosinniederalkylester verwendet wird.

9. Verfahren nach einem der vorangehenden Ansprüche,
**dadurch gekennzeichnet,**
daß man als Aminosäure Alanin, Serin, Glycin, Methionin, Isoleucin, Valin, Leucin oder Arginin, insbesondere Alanin, verwendet.

10. Vorrichtung zur Peptidsynthese nach einem der vorangehenden Ansprüche,
**gekennzeichnet durch**
einen ersten EMR (5), dessen Ausgang abwechselnd mit zwei wechselseitig zu betreibenden Säulen (6) mit hydrophobem Adsorber zu verbinden ist, von denen jeweils die eine als Akkumulationssäule geschaltet ist, deren Ausgang über ein Polarimeter (7) zum EMR-Eingang zurückgelangt, während der Ausgang der anderen mit Elutionsmittel beaufschlagten Säule zu einem weiteren EMR (11) für die Schutzgruppenabspaltung oder zu einer entsprechenden Enzymkolonne führt.

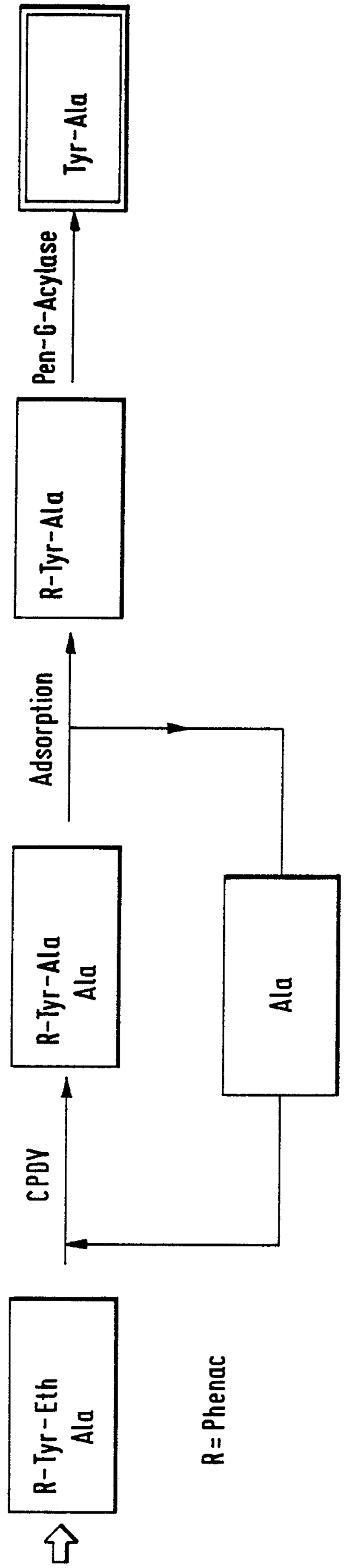
R-Tyr-Eth
Ala
CPDV
R-Tyr-Ala
Ala
Adsorption
R-Tyr-Ala
Pen-G-Acylase
Tyr-Ala
Ala
R=Phenac

FIG. 1

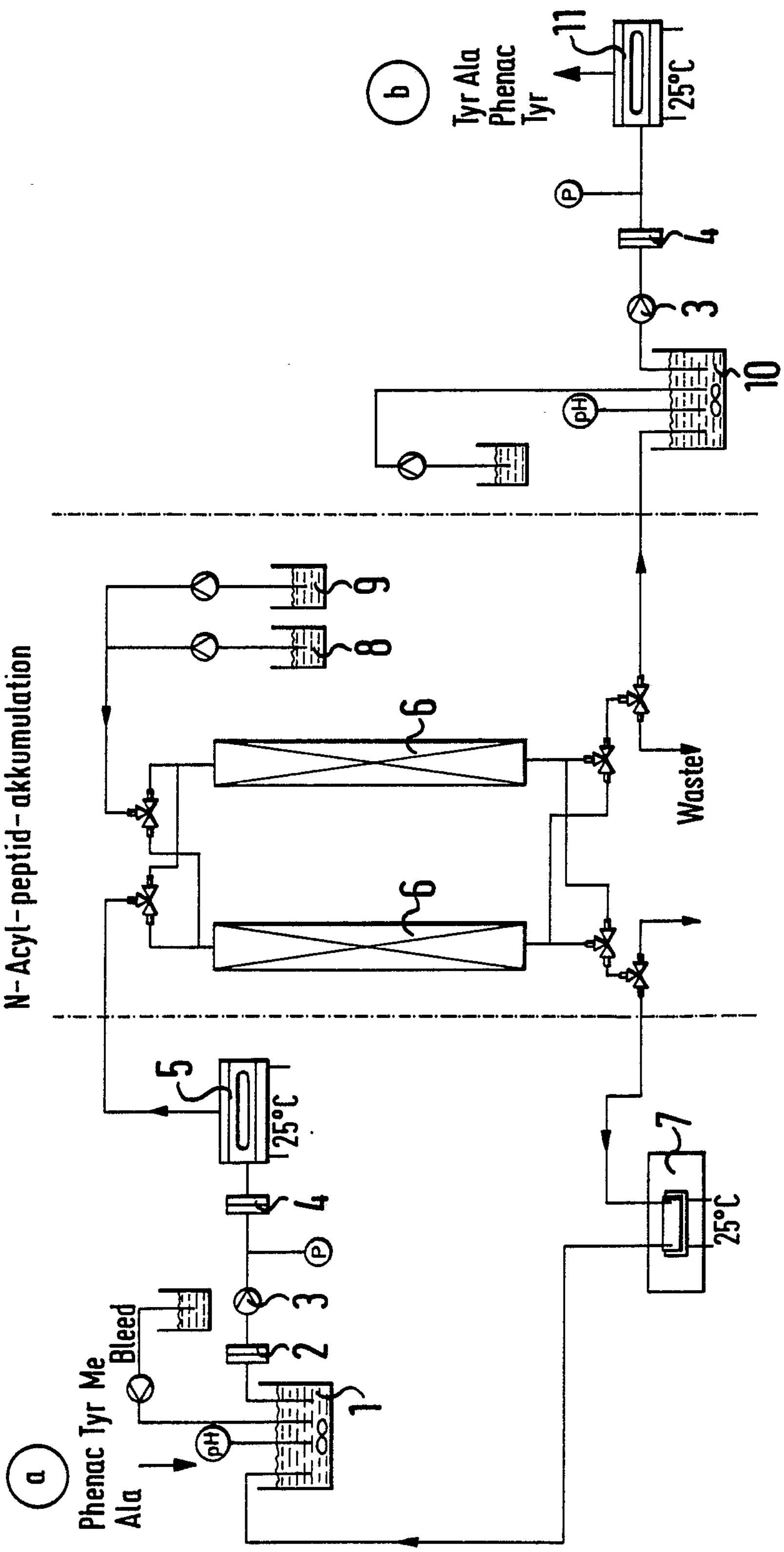
N-Acyl-peptid-akkumulation
a
Phenac Tyr Me
Ala
pH
Bleed
1
2
3
P
4
5
25°C
6
6
7
25°C
8
9
Waste
10
pH
3
4
P
11
25°C
b
Tyr Ala
Phenac
Tyr

FIG. 2